(19) Europäisches Patentamt
European Patent Office
Office européen des brevets

(11) EP 3 279 658 A1

(12) EUROPEAN PATENT APPLICATION

(43) Date of publication:
**07.02.2018 Bulletin 2018/06**

(21) Application number: **17184904.5**

(22) Date of filing: **04.08.2017**

(51) Int Cl.:
*G01N 33/52* (2006.01) *G01N 33/84* (2006.01)
*C12Q 1/26* (2006.01) *C12Q 1/527* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.08.2016 JP 2016154872**
**15.06.2017 JP 2017118044**

(71) Applicant: **ARKRAY, Inc.**
**Minami-ku**
**Kyoto-shi,**
**Kyoto 601-8045 (JP)**

(72) Inventors:
- **Katsuki, Koji**
  **Kyoto, 602-0008 (JP)**
- **Funamoto, Takehiro**
  **Kyoto, 602-0008 (JP)**
- **Murakami, Masaki**
  **Kyoto, 602-0008 (JP)**

(74) Representative: **Hoffmann Eitle**
**Patent- und Rechtsanwälte PartmbB**
**Arabellastraße 30**
**81925 München (DE)**

# (54) QUANTIFICATION METHOD FOR AMMONIA, QUANTIFICATION REAGENT, QUANTIFICATION REAGENT KIT, AND AMMONIA QUANTIFICATION DEVICE

(57) A method of quantifying ammonia, the method includes performing a first reaction in which a test liquid containing ammonia is reacted with adenosine triphosphate and L-glutamic acid in the presence of glutamine synthetase to produce phosphoric acid, performing a second reaction in which the produced phosphoric acid is reacted with pyruvic acid in the presence of pyruvate oxidase, and measuring a component consumed or produced by the second reaction, to quantify ammonia, wherein a reaction to produce adenosine triphosphate from adenosine diphosphate mediated by pyruvate kinase is not carried out.

EP 3 279 658 A1

## Description

### Technical Field

The present invention relates to a quantification method for ammonia, a quantification reagent, a quantification reagent kit, a test piece, and an ammonia quantification device.

### Background Art

Known examples of methods of quantification of ammonia include chemical measurement methods and enzymatic measurement methods. A chemical measurement method that is mainly used is the indophenol method. Examples of the enzymatic measurement methods include methods using glutamate dehydrogenase, as well as methods using glutamine synthetase, carbamate kinase, carbamoyl phosphate synthetase, and/or the like.

Examples of the enzymatic measurement methods using glutamine synthetase that have been proposed include a method in which glutamate synthase is allowed to act in the presence of ammonia, adenosine triphosphate (ATP), and L-glutamic acid, and kinase is allowed to act on the produced ADP and a kinase substrate phosphorus compound, followed by quantifying the produced kinase reaction product to quantify ammonia (see, for example, Patent Document 1).

More specifically, Patent Document 1 discloses a method in which ammonia is quantified by the following four-step reaction.

(1) Glutamine synthetase is allowed to act in the presence of ammonia, ATP, and L-glutamic acid, to produce L-glutamine, adenosine diphosphate (ADP), and phosphoric acid.
(2) Pyruvate kinase is allowed to act in the presence of the produced ADP and phosphoenolpyruvate, to produce ATP and pyruvic acid.
(3) Pyruvate oxidase is allowed to act in the presence of the produced pyruvic acid, produced phosphoric acid, and oxygen, to produce acetyl phosphate, carbon dioxide, and hydrogen peroxide.
(4) Peroxidase is allowed to act in the presence of the produced hydrogen peroxide, phenol, and 4-aminoantipyrine, to produce a quinone pigment. Based on the level of increase in the absorbance at 500 nm, ammonia is quantified.

### Prior Art Document

[Patent Document]

[Patent Document 1] Japanese Patent Application Laid-Open (JP-A) No. S62-3800

## SUMMARY OF THE INVENTION

### Problems to be Solved by the Invention

In the method of quantifying ammonia described in Patent Document 1, ammonia is quantified through a four-step reaction as described above. In a case in which there is a large number of reaction pathways, a large number of reagents are required, which is problematic. Since, in the method of quantifying ammonia described in Patent Document 1, phosphoenolpyruvate, which is expensive, is used, the measurement cost is high, which is problematic. Thus, an inexpensive and simple method of quantifying ammonia has been demanded.

An object of the invention is to provide a quantification method for ammonia, a quantification reagent, a quantification reagent kit, a test piece, and an ammonia quantification device which enable inexpensive and simple quantification of ammonia. Means for Solving the Problems

More specifically, the object can be achieved by the following means.

<1> One embodiment of the invention is a method of quantifying ammonia, the method comprising: performing a first reaction in which a test liquid containing ammonia is reacted with adenosine triphosphate and L-glutamic acid in the presence of glutamine synthetase to produce phosphoric acid; performing a second reaction in which the produced phosphoric acid is reacted with pyruvic acid in the presence of pyruvate oxidase; and measuring a component consumed or produced by the second reaction, to quantify ammonia, wherein a reaction to produce adenosine triphosphate from adenosine diphosphate mediated by pyruvate kinase is not carried out.
<2> One embodiment of the invention is the method of quantifying ammonia according to <1>, wherein the first reaction further includes at least one of a magnesium ion ($Mg^{2+}$) or a manganese ion ($Mn^{2+}$) as a catalyst to produce the phosphoric acid.

<3> One embodiment of the invention is the method of quantifying ammonia according to <1> or <2>, wherein the second reaction further includes the presence of thiamine pyrophosphate and flavin adenine nucleotide as coenzymes.
<4> One embodiment of the invention is the method of quantifying ammonia according to any one of <1> to <3>, wherein the second reaction produces hydrogen peroxide, and the measuring comprises measuring the produced hydrogen peroxide.
<5> One embodiment of the invention is the method of quantifying ammonia according to <4>, wherein the measuring the produced hydrogen peroxide comprises performing a color reaction or an electrochemical reaction.
<6> One embodiment of the invention is a quantifying reagent, comprising pyruvic acid, adenosine triphosphate, L-glutamic acid, and an enzyme, wherein the enzyme consists of glutamine synthetase and pyruvate oxidase.
<7> One embodiment of the invention is a quantifying reagent kit, comprising: a first reagent containing a hydrogen donor; and a second reagent containing a coupler, wherein pyruvic acid, adenosine triphosphate, L-glutamic acid, glutamine synthetase, pyruvate oxidase, and peroxidase are each independently contained in at least one of the first reagent or the second reagent, and wherein the only enzymes in the first and second reagents consist of the glutamine synthetase, the pyruvate oxidase, the peroxidase, or any combination thereof.
<8> One embodiment of the invention is a test piece comprising pyruvic acid, adenosine triphosphate, L-glutamic acid, and an enzyme, wherein the enzyme consists of glutamine synthetase and pyruvate oxidase.
<9> One embodiment of the invention is an ammonia quantification device, performing a first reaction in which a test liquid containing ammonia is reacted with adenosine triphosphate and L-glutamic acid in the presence of glutamine synthetase to produce phosphoric acid; performing a second reaction in which the produced phosphoric acid is reacted with pyruvic acid in the presence of pyruvate oxidase; and measuring a component consumed or produced by the second reaction, to quantify ammonia, wherein a reaction to produce adenosine triphosphate from adenosine diphosphate mediated by pyruvate kinase is not carried out.
<10> One embodiment of the invention is an ammonia quantification device, comprising: a reaction section in which a test subject containing ammonia, pyruvic acid, adenosine triphosphate, L-glutamic acid, glutamine synthetase and pyruvate oxidase, is arranged, wherein, in the test subject, as a first reaction, the ammonia is reacted with the adenosine triphosphate, and the L-glutamic acid in the presence of the glutamine synthetase to produce phosphoric acid, and, as a second reaction, the produced phosphoric acid is reacted with the pyruvic acid in the presence of the pyruvate oxidase; and a measurement section in which a component consumed or produced in the reaction section, is measured, wherein a reaction to produce adenosine triphosphate from adenosine diphosphate mediated by pyruvate kinase is not carried out in the reaction section.
<11> One embodiment of the invention is the ammonia quantification device according to <10>, wherein: the test subject further contains peroxidase, a hydrogen donor, and a coupler; in the reaction section: the second reaction produces hydrogen peroxide, and as a third reaction, the produced hydrogen peroxide is reacted with the hydrogen donor and the coupler in the presence of the peroxidase to produce a pigment; and in the measurement section, the pigment produced in the reaction section is measured.

According to one embodiment of the invention, a quantification method for ammonia, a quantification reagent, a quantification reagent kit, a test piece and an ammonia quantification device that enable inexpensive and simple quantification of ammonia can be provided.

BRIEF DESCRIPTION OF THE DRAWINGS

Fig. 1 is a graph showing the relationship between the amount of ammonia and the amount of increase in the absorbance at 560 nm in Example 1.
Fig. 2 is a graph showing the relationship between the concentration of ammonia and the K/S value in Example 2.
Fig. 3 is a schematic configuration diagram illustrating a quantification device 100 according to one embodiment of the invention.

MODE FOR CARRYING OUT THE INVENTION

A quantification method for ammonia, a quantification reagent, a quantification reagent kit, a test piece and an ammonia quantification device as one embodiment of the invention are described below.
In the present description, each numerical range expressed using "from ... to ..." means the range including the values described before and after "to" as the lower limit and the upper limit, respectively.
In the present description, "test liquid containing ammonia'' means a test liquid containing at least one of

ammonia or ammonium ion, and "ammonia concentration" and "amount of ammonia" mean the total concentration and the total amount, respectively, of ammonia and ammonium ion.

In the present description, each of the L-glutamic acid, phosphoric acid, pyruvic acid, and acetyl phosphate may be a salt.

[Method of Quantifying Ammonia]

One embodiment of the invention is a method of quantifying ammonia, the method comprising: performing a first reaction in which a test liquid containing ammonia is reacted with adenosine triphosphate (ATP) and L-glutamic acid in the presence of glutamine synthetase to produce phosphoric acid; performing a second reaction in which the produced phosphoric acid is reacted with pyruvic acid in the presence of pyruvate oxidase; and measuring a component consumed or produced by the second reaction, to quantify ammonia, wherein a reaction to produce adenosine triphosphate from adenosine diphosphate (ADP) mediated by pyruvate kinase is not carried out.

In the quantification method of the present embodiment, ammonia (the total of ammonia and ammonium ion) contained in the test liquid can be quantified by measuring a component consumed or produced through a two-step reaction including a reaction mediated by glutamine synthetase (the first reaction) and a reaction mediated by pyruvate oxidase (the second reaction).

In conventionally known methods of quantifying ammonia, a reaction mediated by pyruvate kinase is carried out in addition to a reaction mediated by glutamine synthetase and a reaction mediated by pyruvate oxidase. Thus, ammonia contained in a test liquid is quantified through a reaction including at least three steps. More concretely, in the reaction mediated by pyruvate kinase, ADP produced by a reaction mediated by glutamine synthetase is reacted with phosphoenolpyruvate in the presence of pyruvate kinase, to produce ATP and the pyruvic acid to be used in the reaction mediated by pyruvate oxidase. In conventionally known methods of quantifying ammonia, a reaction mediated by pyruvate kinase is carried out as part of the cycling reaction of ATP and ADP.

However, in conventionally known methods of quantifying ammonia, a reaction using phosphoenolpyruvate is required as part of the cycling reaction of ATP and ADP. Since phosphoenolpyruvate is expensive compared to ATP, pyruvic acid, and the like, the measurement cost is high, which is problematic. Moreover, since it is assumed that a reaction to allow pyruvate oxidase to act using a rather large amount of phosphoenolpyruvate not to be used for the cycling may be stably carried out from the viewpoint of favorably carrying out the quantification of ammonia, the measurement cost may be even higher.

The present embodiment is a method of quantifying ammonia in which a reaction to produce ATP from ADP mediated by pyruvate kinase is not carried out. Therefore, since the method of quantifying ammonia of the present embodiment has a smaller number of reaction pathways than conventionally known methods of quantifying ammonia, the number of reagents required can be reduced. Thus, by the quantification method of the present embodiment, ammonia can be more simply quantified. Moreover, since the quantification method of the present embodiment does not use phosphoenolpyruvate, which is expensive, ammonia can be quantified at a lower cost.

In the quantification method of the present embodiment, a test liquid containing ammonia is used. The test liquid is not limited as long as the test liquid contains ammonia. Examples of the test liquid include test liquids containing ammonia produced by an enzymatic reaction, and test liquids containing ammonia produced or released by a chemical reaction (for example, hydrolysis). More specific examples of the test liquid include blood, serum, and urine. In the quantification method of the present embodiment, the quantification of ammonia may be carried out by adding the later-described components to the test liquid to be analyzed, or the quantification of ammonia may be carried out by attaching the test liquid containing ammonia to filter paper or the like impregnated with the later-described components.

In the quantification method of the present embodiment, a test liquid containing ammonia is reacted with ATP and L-glutamic acid in the presence of glutamine synthetase, to produce phosphoric acid (the first reaction). For example, a test liquid containing ammonia ($NH_3$) is reacted with ATP and L-glutamate in the presence of glutamine synthetase. As a result, ADP, orthophosphate, and L-glutamine are produced as shown in the following Reaction Formula (1).

$$NH_3 + \text{L-Glutamate} + \text{ATP} \xrightarrow{\text{Glutamine Synthetase}} \text{ADP} + \text{Orthophosphate} + \text{L-Glutamine} \quad (1)$$

In the quantification method of the present embodiment, from the viewpoint of efficiently carrying out the synthesis reaction of phosphoric acid, it is preferred that the first reaction further include at least one of a magnesium ion ($Mg^{2+}$) or a manganese ion ($Mn^{2+}$) as a catalyst to produce the phosphoric acid.

In the quantification method of the present embodiment, the produced phosphoric acid is reacted with pyruvic acid in the presence of pyruvate oxidase (the second reaction). For example, the produced orthophosphate is reacted

with pyruvate, and oxygen (in an aqueous solution containing) in the presence of pyruvate oxidase. As a result, as shown in the following Reaction Formula (2), acetyl phosphate, carbon dioxide, and hydrogen peroxide are produced.

$$\text{Pyruvate} + \text{Orthophosphate} + O_2 + H_2O \xrightarrow{\text{Pyruvate Oxidase}} \text{Acetyl phosphate} + CO_2 + H_2O_2 \qquad (2)$$

The produced phosphoric acid is reacted with pyruvic acid in the presence of pyruvate oxidase, and a component consumed or produced by the second reaction is measured to quantify ammonia. For example, the quantification of ammonia may be carried out by measuring the oxygen consumed in the second reaction, or the quantification of ammonia may be carried out by measuring the hydrogen peroxide produced in the second reaction.

In a case in which ammonia is quantified by measuring the consumed oxygen, for example, it is preferred to measure the dissolved oxygen level in the solution using an oxygen electrode.

In a case in which ammonia is quantified by measuring the produced hydrogen peroxide, for example, it is preferred to carry out a color reaction or an electrochemical reaction.

In a case in which the color reaction is carried out, for example, the produced hydrogen peroxide is reacted with a hydrogen donor and a coupler in the presence of the peroxidase (a third reaction). As a result, a pigment is produced by oxidative condensation reaction of the hydrogen donor and the coupler. Since the absorbance at a particular wavelength absorbed by the produced pigment is in a proportional relationship with the concentration of ammonia contained in the test liquid, the ammonia can be quantified by measuring the absorbance at the particular wavelength.

Alternatively, the quantification of ammonia may be carried out by measuring the reflectance of test paper obtained by attaching the produced pigment on filter paper or the like. For example, the quantification of ammonia may be carried out by measuring the reflectance of test paper obtained by impregnating filter paper or the like with a liquid containing the produced hydrogen peroxide, a hydrogen donor, and peroxidase, and with a liquid containing a coupler.

For example, the produced hydrogen peroxide is reacted with a hydrogen donor disodium 1-naphthol-3,6-disulfonate (NDSA), and the coupler 4-aminoantipyrine in the presence of the peroxidase. As a result, as shown in the following Reaction Formula (3), a quinoneimine dye is produced to increase the absorbance at 560 nm.

$$H_2O_2 + \text{NDSA} + \text{4-Aminoantipyrine} \xrightarrow{\text{Peroxidase}} \text{Quinoneimine dye} + 4H_2O \qquad (3)$$

The hydrogen donor is not limited as long as the hydrogen donor causes production of a pigment by oxidative condensation reaction with a coupler, and examples of the hydrogen donor include phenol, phenol derivatives, naphthol, naphthol derivatives, aniline, aniline derivatives, naphthylamine, and naphthylamine derivatives. In particular, aniline or an aniline derivative is preferred from the viewpoint of the sensitivity and the wavelength for the pigment obtained.

Examples of the aniline derivatives include N-ethyl-N-sulfopropyl-3-methoxyaniline, N-ethyl-N-sulfopropylaniline, N-ethyl-N-sulfopropyl-3-methylaniline, N-ethyl-N-(2-hydroxy-3-sulfopropyl)-3-methoxyaniline, N-ethyl-N-(2-hydroxy-3-sulfopropyl)-3,5-dimethoxyaniline, N-(2-hydroxy-3-sulfopropyl)-3,5-dimethoxyaniline, and N-(2-hydroxy-3-sulfopropyl)-2,5-dimethylaniline.

The coupler is not limited as long as the coupler causes production of a pigment by oxidative condensation reaction with a hydrogen donor, and examples of the coupler include 4-aminoantipyrine, 4-aminoantipyrine derivatives, phenylenediamine, and 3-methyl-2-benzothiazolinone hydrazone (MBTH).

In a case in which an electrochemical reaction is carried out, for example, the value of the electric current generated by the electrochemical reaction of the produced hydrogen peroxide may be measured. Since the measured electric current value is in a proportional relationship with the concentration of ammonia contained in the test liquid, the ammonia can be quantified by measuring the electric current value.

In the quantification method of the present embodiment, it is preferred that the second reaction further include the presence of thiamine pyrophosphate and flavin adenine nucleotide as coenzymes for pyruvate oxidase.

In the quantification method of the present embodiment, the reaction temperature is preferably from 10°C to 50°C, more preferably from 20°C to 40°C, still more preferably from 30°C to 37°C. The reaction time is preferably from 1 minute to 60 minutes, more preferably from 2 minutes to 30 minutes, still more preferably from 5 minutes to 15 minutes.

In the quantification method of the present embodiment, a buffer may be used for adjusting the pH of the test liquid containing ammonia to a pH suitable for enzymatic reaction (for example, to a pH of from 6.0 to 9.0). Examples of the pH of the buffer that may be used include pHs of preferably from 6.0 to 9.0, more preferably from 6.0 to 8.0. Examples of the buffer include Good's buffers such as N,N-bis(2-hydroxyethyl)-2-aminoethanesulfonic acid (BES); phosphate buffer; imidazole acid buffer; Tris buffer; and glycine buffer.

In the quantification method of the present embodiment, a component(s) other than the components described

above may be added to the test liquid containing ammonia, if necessary. Examples of the other component(s) include surfactants, antiseptics, and stabilizers.

[Quantification Reagent, Quantification Reagent Kit and Test Piece]

One embodiment of the invention is a quantifying reagent, comprising pyruvic acid, ATP, L-glutamic acid, and an enzyme, wherein the enzyme consists of glutamine synthetase and pyruvate oxidase. The quantifying reagent of the present embodiment is used for, for example, quantification of ammonia.

Another embodiment of the invention is a quantification reagent kit, comprising: a first reagent containing a hydrogen donor; and a second reagent containing a coupler; wherein pyruvic acid, ATP, L-glutamic acid, and, glutamine synthetase, pyruvate oxidase, and peroxidase, are each independently contained in at least one of the first reagent or the second reagent; and wherein the only enzymes in the first and second reagents consist of the glutamine synthetase, the pyruvate oxidase, the peroxidase, or any combination thereof. The quantifying reagent kit of the present embodiment is used for, for example, quantification of ammonia.

One embodiment of the invention is a test piece including pyruvic acid, adenosine triphosphate, L-glutamic acid, and an enzyme, wherein the enzyme consists of glutamine synthetase and pyruvate oxidase. The test piece of the present embodiment is used for, for example, quantification of ammonia.

In the quantification reagent, the quantification reagent kit and the test piece of the present embodiment, ammonia (the total of ammonia and ammonium ion) contained in a test liquid can be quantified by measuring a component consumed or produced through a two-step reaction including a reaction mediated by glutamine synthetase and a reaction mediated by pyruvate oxidase.

By using the quantification reagent, the quantification reagent kit and the test piece of the present embodiment, ammonia can be quantified without carrying out a reaction to produce ATP from ADP mediated by pyruvate kinase. Thus, by the quantification reagent ,the quantification reagent kit and the test piece of the present embodiment, ammonia can be more simply quantified.

Moreover, by the produced hydrogen peroxide being reacted with a hydrogen donor and a coupler in the presence of the peroxidase using the quantification reagent kit of the present embodiment, oxidative condensation reaction of the hydrogen donor and the coupler can be allowed to occur, resulting in production of a pigment, so that quantification of ammonia can be carried out in the same manner as in the quantification method of the present embodiment.

The quantification reagent, the test piece and the first reagent and the second reagent in the quantification reagent kit, of the present embodiment may contain the catalyst, the coenzyme, the buffer and/or the other component described in the method of quantifying ammonia. Since the hydrogen donor and the coupler in the quantification reagent kit of the present embodiment are the same as the hydrogen donor and the coupler described in the method of quantifying ammonia, description of the hydrogen donor and the coupler is omitted.

The test piece of the present embodiment may further include, for example, a hydrogen donor and a coupler, may be filter paper or the like, and/or may be filter paper or the like impregnated with the components.

[Ammonia Quantification Device]

One embodiment of the invention is an ammonia quantification device, performing a first reaction in which a test liquid containing ammonia is reacted with adenosine triphosphate and L-glutamic acid in the presence of glutamine synthetase to produce phosphoric acid; performing a second reaction in which the produced phosphoric acid is reacted with pyruvic acid in the presence of pyruvate oxidase; and measuring a component consumed or produced by the second reaction, to quantify ammonia, wherein a reaction to produce adenosine triphosphate from adenosine diphosphate mediated by pyruvate kinase is not carried out.

In the quantification device of the present embodiment, ammonia (the total of ammonia and ammonium ion) contained in the test liquid can be quantified by measuring a component consumed or produced through a two-step reaction including a reaction mediated by glutamine synthetase and a reaction mediated by pyruvate oxidase.

By using the quantification device of the present embodiment, ammonia can be quantified without carrying out a reaction to produce ATP from ADP mediated by pyruvate kinase. Thus, ammonia can be more simply quantified with the quantification device of the present embodiment.

Specific examples of the quantification device of the present embodiment are described below. Specific Example 1 of the quantification device of the present embodiment is a quantification device including: a reaction section in which a test subject containing ammonia, pyruvic acid, adenosine triphosphate, L-glutamic acid, glutamine synthetase and pyruvate oxidase, is arranged, wherein, in the test subject, as a first reaction, the ammonia is reacted with the adenosine triphosphate, and the L-glutamic acid in the presence of the glutamine synthetase to produce phosphoric acid, and, as a second reaction, the produced phosphoric acid is reacted with the pyruvic acid in the presence of the pyruvate oxidase;

and a measurement section in which a component consumed or produced in the reaction section, is measured. For example, as shown in Fig. 1, a quantification device 100 according to one embodiment of the invention may have at least a reaction section 1 for carrying out the reaction described above and a measurement section 2 for measuring a component consumed or produced by the reaction in the reaction section 1.

The quantification device of Specific Example 1 has the reaction section described above. The reaction section has a constitution in which the test subject can be arranged. For example, the reaction section may have a constitution having the following: a place where a blood collection tube, urine collection tube, or the like storing a test liquid to be tested is arranged; a container storing a test liquid; filter paper which is impregnated with components to be used for the quantification of ammonia and to which a test liquid containing ammonia is attached; or the like.

The reaction section preferably has a constitution with which a reaction condition(s) (reaction temperature, pH of the test liquid, and/or the like) can be controlled such that the reaction producing phosphoric acid shown in the Reaction Formula (1) and the reaction producing hydrogen peroxide shown in the Reaction Formula (2) can be allowed to proceed efficiently.

The quantification device of Specific Example 1 has the measurement section in which a component consumed or produced by the reaction in the reaction section is measured. In the measurement section, ammonia is quantified by, for example, measuring oxygen consumed by the reaction in the reaction section or hydrogen peroxide produced by the reaction in the reaction section.

In a case where oxygen consumed by the reaction in the reaction section is measured in the measurement section, the measurement section preferably has a constitution having an oxygen electrode in which the dissolved oxygen level in a solution is measured using the oxygen electrode.

In a case where hydrogen peroxide produced by the reaction in the reaction section is measured in the measurement section, the measurement section preferably has a constitution in which the value of the electric current generated by the electrochemical reaction of the produced hydrogen peroxide is measured.

Specific Example 2 of the quantification device of the present embodiment is an ammonia quantification device, including:

> a reaction section in which a test subject containing ammonia, pyruvic acid, adenosine triphosphate, L-glutamic acid, an enzyme consisting of glutamine synthetase, pyruvate oxidase, and peroxidase, a hydrogen donor, and a coupler is arranged, wherein in the test subject, as a first reaction, the ammonia is reacted with the adenosine triphosphate, and the L-glutamic acid in the presence of the glutamine synthetase to produce phosphoric acid, and, as a second reaction, the produced phosphoric acid is reacted with the pyruvic acid in the presence of the pyruvate oxidase; and as a third reaction, the produced hydrogen peroxide is reacted with the hydrogen donor and the coupler in the presence of the peroxidase to produce a pigment; and a measurement section in which the pigment produced by the reaction in the reaction section is measured.

The measurement section for measuring the pigment is not limited as long as the measurement section has a constitution in which the absorbance at a particular wavelength absorbed by the pigment is measured, a constitution in which the reflectance of a test paper to which the pigment is attached is measured, or the like.

The test subject containing ammonia to be analyzed in the quantification device of the present embodiment may also contain the catalyst, the coenzyme, the buffer, and/or the other component described above in the method for quantifying ammonia.

The test subject containing ammonia and the components described above may be provided in advance, and the quantification device of the present embodiment may have a constitution in which the provided test subject is arranged in the reaction section.

The quantification device of the present embodiment may further have a storage section for storing the quantification reagent, for storing the first reagent and the second reagent, or for storing the components described above, and may have a constitution in which the reagents or components are supplied to the test liquid containing ammonia arranged in the reaction section. Alternatively, the quantification device of the present embodiment may have a constitution in which a test liquid containing ammonia is attached to filter paper or the like impregnated with the components to be used for the quantification of ammonia, arranged in the reaction section.

## EXAMPLES

One embodiment of the invention is described below by way of Examples, but the invention is not limited to the Examples.

[Example 1]

The Reagent 1 and the Reagent 2 described below were prepared. As the Reagent 2, four kinds of reagents having different concentrations of ammonium sulfate were prepared.

<Reagent 1>

BES - sodium hydroxide buffer (manufactured by Dojindo Molecular Technologies, Inc.; pH 7.0) 100 mmol/L
Sodium L-glutamate (manufactured by Nacalai Tesque, Inc.) 15 mmol/L
Disodium 1-naphthol-3,6-disulfonate (manufactured by Wako Pure Chemical Industries, Ltd.) 5 mmol/L
Thiamine pyrophosphate (manufactured by Tokyo Chemical Industry Co., Ltd.) 1 mmol/L
Flavin adenine dinucleotide (manufactured by Tokyo Chemical Industry Co., Ltd.) 1 mmol/L
Magnesium chloride hexahydrate 20 mmol/L
Adenosine triphosphate dihydrate (manufactured by Roche) 15 mmol/L
Potassium pyruvate (manufactured by Nacalai Tesque, Inc.) 15 mmol/L
Glutamine synthetase (manufactured by Kikkoman Co., Ltd.) 10 U/mL
Peroxidase (manufactured by Toyobo Co., Ltd.) 10 U/mL

<Reagent 2>

4-Aminoantipyrine (manufactured by Wako Pure Chemical Industries, Ltd.) 5 mmol/L
Pyruvate oxidase (manufactured by Asahi Kasei Corporation) 10 U/mL
Ammonium sulfate (manufactured by Nacalai Tesque, Inc.) 0 mmol/L, 1.8 mmol/L, 3.6 mmol/L, 7.2 mmol/L

Subsequently, 200 μL of each Reagent 2 was mixed with 800 μL of Reagent 1, and the reaction was allowed to proceed at 37°C for 10 minutes. Subsequently, the absorbance of the reagent mixture was measured. As a result, as shown in Fig. 1, a favorable linear relationship could be obtained between the amount of ammonia in Reagent 2 and the amount of increase in the absorbance at 560 nm.

[Example 2]

The following Impregnation Liquid 1 and Impregnation Liquid 2 were prepared, and filter paper was provided.

<Impregnation Liquid 1>

BES-sodium hydroxide buffer (manufactured by Dojindo Molecular Technologies, Inc.; pH 7.5) 150 mmol/L
Sodium L-glutamate (manufactured by Nacalai Tesque, Inc.) 150 mmol/L
Disodium 1-naphthol-3,6-disulfonate (manufactured by Wako Pure Chemical Industries, Ltd.) 10 mmol/L
Thiamine pyrophosphate (manufactured by Tokyo Chemical Industry Co., Ltd.) 1.5 mmol/L
Flavin adenine dinucleotide (manufactured by Tokyo Chemical Industry Co., Ltd.) 1.5 mmol/L
Magnesium chloride hexahydrate 50 mmol/L
Adenosine triphosphate dihydrate (manufactured by Roche) 15 mmol/L
Potassium pyruvate (manufactured by Nacalai Tesque, Inc.) 30 mmol/L
Glutamine synthetase (manufactured by Kikkoman Co., Ltd.) 45 U/mL
Pyruvate oxidase (manufactured by Asahi Kasei Corporation) 70 U/mL
Peroxidase (manufactured by Toyobo Co., Ltd.) 70 U/mL

<Impregnation Liquid 2>

4-Aminoantipyrine (manufactured by Wako Pure Chemical Industries, Ltd.) 15 mmol/L

<Filter Paper>

3MM HP (manufactured by Whatman)

Subsequently, the filter paper was impregnated with Impregnation Liquid 1 and Impregnation Liquid 2, and then dried to obtain a test piece. On the obtained test piece, aqueous ammonium sulfate solutions having the ammonia concentrations shown in Fig. 2 were spotted, and the test piece was left to stand at normal temperature for 10 minutes, followed by reading the reflectance (560 nm) of the position on which each aqueous ammonium sulfate solution was spotted, and converting the reflectance to the following K/S value.

$$K/S \text{ value} = (1-R)^2/2R \text{ (Kubelka-Munk equation; R represents the reflectance)}$$

(Kubelka-Munk equation; R represents the reflectance)

As shown in Fig. 2, a favorable linear relationship could be obtained between the ammonia concentration and the K/S value at 560 nm.

Thus, the present Examples showed that highly sensitive quantification of ammonia is possible by a simple method.

DESCRIPTION OF SYMBOLS

1. Reaction section
2. Measurement section
100. Quantification device

## Claims

1. A method of quantifying ammonia, the method comprising:

    performing a first reaction in which a test liquid containing ammonia is reacted with adenosine triphosphate and L-glutamic acid in the presence of glutamine synthetase to produce phosphoric acid;
    performing a second reaction in which the produced phosphoric acid is reacted with pyruvic acid in the presence of pyruvate oxidase; and
    measuring a component consumed or produced by the second reaction, to quantify ammonia,
    wherein a reaction to produce adenosine triphosphate from adenosine diphosphate mediated by pyruvate kinase is not carried out.

2. The method of quantifying ammonia according to claim 1, wherein the first reaction further includes at least one of a magnesium ion ($Mg^{2+}$) or a manganese ion ($Mn^{2+}$) as a catalyst to produce the phosphoric acid.

3. The method of quantifying ammonia according to claim 1 or 2, wherein the second reaction further includes the presence of thiamine pyrophosphate and flavin adenine nucleotide as coenzymes.

4. The method of quantifying ammonia according to any one of claims 1 to 3, wherein the second reaction produces hydrogen peroxide, and the measuring comprises measuring the produced hydrogen peroxide.

5. The method of quantifying ammonia according to claim 4, wherein the measuring the produced hydrogen peroxide comprises performing a color reaction or an electrochemical reaction.

6. A quantifying reagent, comprising pyruvic acid, adenosine triphosphate, L-glutamic acid, and an enzyme, wherein the enzyme consists of glutamine synthetase and pyruvate oxidase.

7. A quantifying reagent kit, comprising:

    a first reagent containing a hydrogen donor; and

a second reagent containing a coupler,
wherein pyruvic acid, adenosine triphosphate, L-glutamic acid, glutamine synthetase, pyruvate oxidase, and peroxidase are each independently contained in at least one of the first reagent or the second reagent, and
wherein the only enzymes in the first and second reagents consist of the glutamine synthetase, the pyruvate oxidase, the peroxidase, or any combination thereof.

**8.** A test piece comprising pyruvic acid, adenosine triphosphate, L-glutamic acid, and an enzyme, wherein the enzyme consists of glutamine synthetase and pyruvate oxidase.

**9.** An ammonia quantification device, comprising:

a reaction section in which a test subject containing ammonia, pyruvic acid, adenosine triphosphate, L-glutamic acid, glutamine synthetase and pyruvate oxidase, is arranged, wherein, in the test subject, as a first reaction, the ammonia is reacted with the adenosine triphosphate, and the L-glutamic acid in the presence of the glutamine synthetase to produce phosphoric acid, and, as a second reaction, the produced phosphoric acid is reacted with the pyruvic acid in the presence of the pyruvate oxidase; and
a measurement section in which a component consumed or produced in the reaction section, is measured.

**10.** The ammonia quantification device according to claim 9, wherein:

the test subject further contains peroxidase, a hydrogen donor, and a coupler;
in the reaction section:

the second reaction produces hydrogen peroxide, and
as a third reaction, the produced hydrogen peroxide is reacted with the hydrogen donor and the coupler in the presence of the peroxidase to produce a pigment; and

in the measurement section, the pigment produced in the reaction section is measured.

Fig. 1

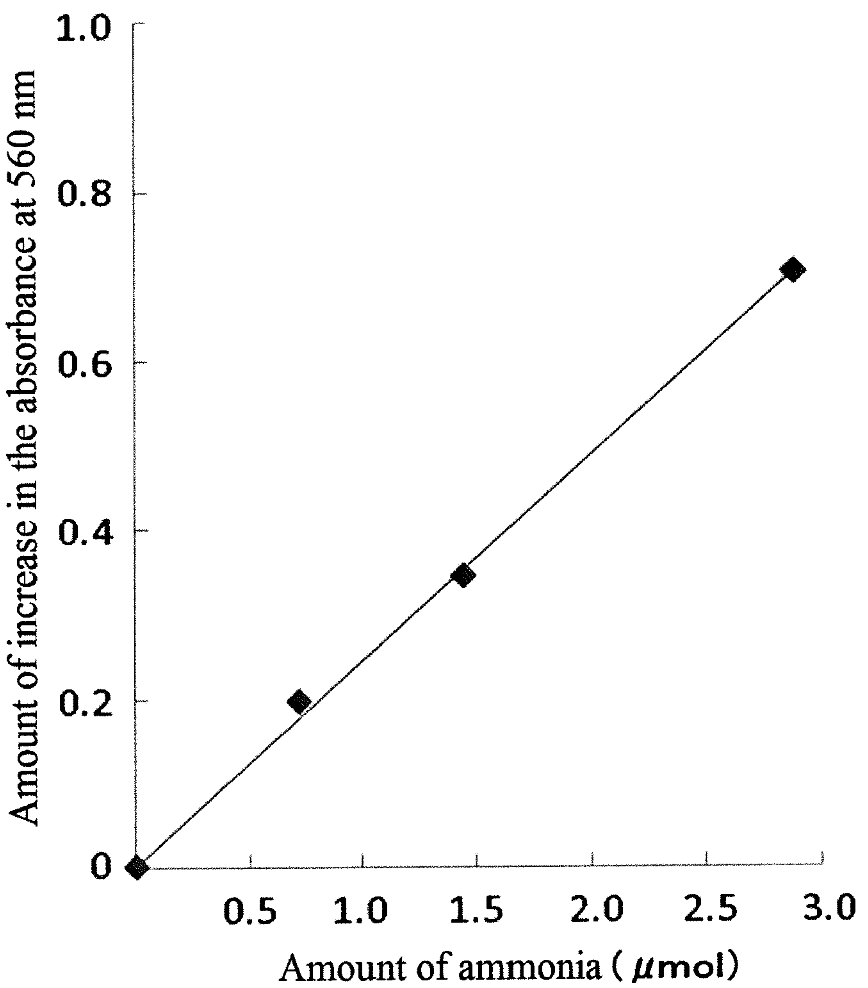
1.0
0.8
0.6
0.4
0.2
0
Amount of increase in the absorbance at 560 nm
0.5
1.0
1.5
2.0
2.5
3.0
Amount of ammonia (μmol)

Fig. 2

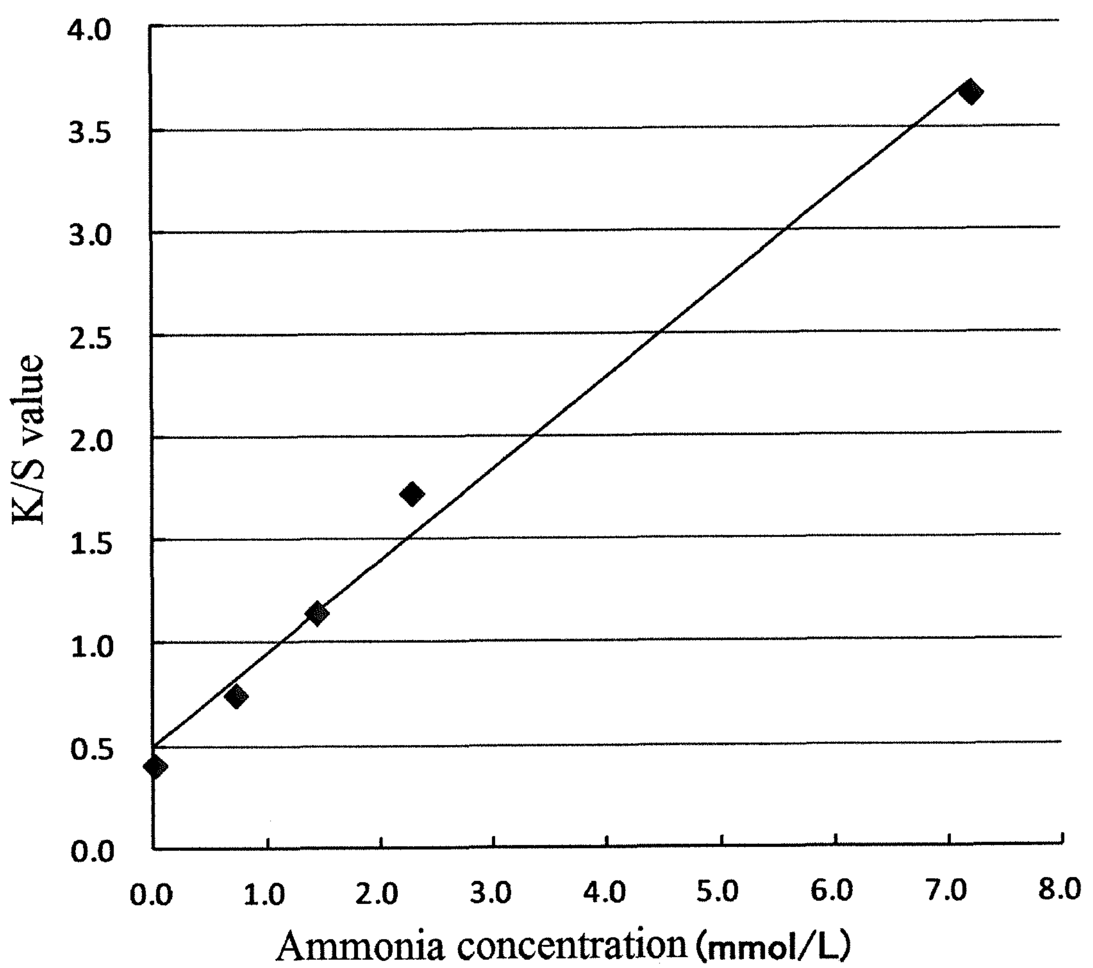
4.0
3.5
3.0
2.5
2.0
1.5
1.0
0.5
0.0
K/S value
0.0
1.0
2.0
3.0
4.0
5.0
6.0
7.0
8.0
Ammonia concentration (mmol/L)

Fig. 3

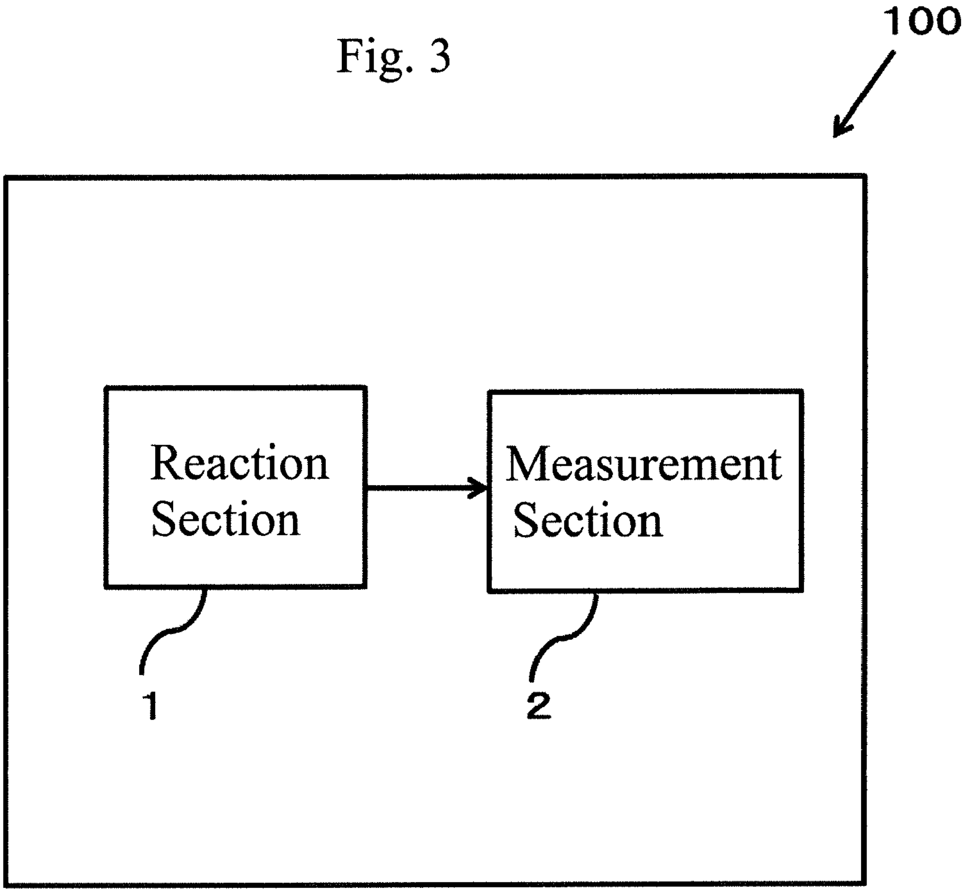

Europäisches Patentamt
European Patent Office
Office européen des brevets

# EUROPEAN SEARCH REPORT

Application Number
EP 17 18 4904

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 1 749 755 A (WANG ERZHONG [CN]) 22 March 2006 (2006-03-22)<br>* abstract *<br>* paragraph [0011] - paragraph [0018] *<br>* paragraph [0024] - paragraph [0031] *<br>* paragraph [0057] - paragraph [0074] *<br>* paragraph [0034] - paragraph [0056] *<br>* claim 1 *<br>----- | 1-10 | INV.<br>G01N33/52<br>G01N33/84<br>C12Q1/26<br>C12Q1/527 |
| X | CN 1 769 475 A (WANG ERZHONG [CN]) 10 May 2006 (2006-05-10)<br>* abstract *<br>* paragraph [0012] *<br>* paragraph [0016] *<br>* paragraph [0020] - paragraph [0101] *<br>* paragraph [0117] *<br>----- | 1-10 | |
| X | CN 1 778 945 A (AIJIE BIOLOG SCIENCE AND TECHN [CN]) 31 May 2006 (2006-05-31)<br>* abstract *<br>* paragraph [0011] - paragraph [0018] *<br>* paragraph [0146] *<br>* claims 1-9 *<br>----- | 1,6,9 | TECHNICAL FIELDS SEARCHED (IPC)<br>G01N<br>C12Q |
| X | CN 1 749 756 A (WANG ERZHONG [CN]) 22 March 2006 (2006-03-22)<br>* abstract *<br>* paragraph [0010] - paragraph [0018] *<br>* paragraph [0103] *<br>----- | 1,6,9 | |
| X | JP H03 91497 A (FUJI PHOTO FILM CO LTD) 17 April 1991 (1991-04-17)<br>* abstract *<br>* page 2 of the english translation, lines 6-28 *<br>----- | 6,8 | |

The present search report has been drawn up for all claims

1

| Place of search | Date of completion of the search | Examiner |
|---|---|---|
| Munich | 6 October 2017 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 18 4904

This annex lists the patent family members relating to the patent documents cited in the above-mentioned European search report. The members are as contained in the European Patent Office EDP file on
The European Patent Office is in no way liable for these particulars which are merely given for the purpose of information.

06-10-2017

| Patent document cited in search report | Publication date | Patent family member(s) | Publication date |
|---|---|---|---|
| CN 1749755 A | 22-03-2006 | NONE | |
| CN 1769475 A | 10-05-2006 | NONE | |
| CN 1778945 A | 31-05-2006 | NONE | |
| CN 1749756 A | 22-03-2006 | NONE | |
| JP H0391497 A | 17-04-1991 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S623800 A